(19) Europäisches Patentamt

European Patent Office

Office européen des brevets

(11) **EP 1 986 990 B1**

(12) **EUROPÄISCHE PATENTSCHRIFT**

(45) Veröffentlichungstag und Bekanntmachung des Hinweises auf die Patenterteilung:
**14.08.2013  Patentblatt 2013/33**

(51) Int Cl.:
***C07C 209/16*** (2006.01)    ***C07C 211/10*** (2006.01)
***C07C 211/14*** (2006.01)

(21) Anmeldenummer: **07712170.5**

(22) Anmeldetag: **08.02.2007**

(86) Internationale Anmeldenummer:
**PCT/EP2007/051210**

(87) Internationale Veröffentlichungsnummer:
**WO 2007/093552 (23.08.2007 Gazette 2007/34)**

(54) **VERFAHREN ZUR HERSTELLUNG VON ETHYLENAMINEN UND ETHANOLAMINEN DURCH HYDRIERENDE AMINIERUNG VON MONOETHYLENGLYKOL UND AMMONIAK IN GEGENWART EINES KATALYSATORS**

METHOD FOR PRODUCING ETHYLENE AMINES AND ETHANOL AMINES BY THE HYDROGENATING AMINATION OF MONOETHYLENE GLYCOL AND AMMONIA IN THE PRESENCE OF A CATALYST

PROCÉDÉ POUR PRODUIRE DES ÉTHYLÈNEAMINES ET DES ÉTHANOLAMINES PAR HYDROAMINATION DE MONOÉTHYLÈNEGLYCOL ET D'AMMONIAC EN PRÉSENCE D'UN CATALYSEUR

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **14.02.2006  EP 06101642**

(43) Veröffentlichungstag der Anmeldung:
**05.11.2008  Patentblatt 2008/45**

(73) Patentinhaber: **BASF SE**
**67056 Ludwigshafen (DE)**

(72) Erfinder:
• **CAUWENBERGE, Gunther van**
**B-9140 Temse (BE)**
• **MELDER, Johann-Peter**
**67459 Böhl-Iggelheim (DE)**
• **HOFFER, Bram, Willem**
**69120 Heidelberg (DE)**
• **KRUG, Thomas**
**67550 Worms (DE)**
• **PICKENÄCKER, Karin**
**68623 Lampertheim (DE)**
• **PAPE, Frank-Friedrich**
**67259 Kleinniedesheim (DE)**
• **SCHWAB, Ekkehard**
**67434 Neustadt (DE)**

(56) Entgegenhaltungen:
**EP-A- 0 839 575     US-A- 4 855 505**

Bemerkungen:
Die Akte enthält technische Angaben, die nach dem Eingang der Anmeldung eingereicht wurden und die nicht in dieser Patentschrift enthalten sind.

EP 1 986 990 B1

**Beschreibung**

Die Erfindung betrifft ein Verfahren zur Herstellung von Ethylenaminen und Ethanolaminen durch hydrierende Aminierung von Monoethylenglykol (im Folgenden: MEG) und Ammoniak in Gegenwart eines Katalysators.

In bekannten Verfahren wird in der Regel ein Gemisch an Ethanolaminen und Ethylenaminen erhalten; hiervon sind insbesondere Ethylendiamin (im Folgenden: EDA) und Diethylentriamin (Bis(2-aminoethyl)amin; im Folgenden: DETA), wichtige Wertstoffe, unter anderem zur Verwendung als Lösungsmittel, Stabilisatoren, zur Synthese von Chelat-Bildnern, Kunstharzen, Arzneimitteln, Inhibitoren und grenzflächenaktiven Substanzen.

EDA wird insbesondere als Rohstoff für Fungizide und Insektizide eingesetzt.

DETA findet insbesondere Verwendung als Lösungsmittel für Farbstoffe und ist Ausgangsmaterial zur Herstellung von Ionenaustauschern, Schädlingsbekämpfungsmitteln, Antioxidantien, Korrosionsschutzmitteln, Komplexbildnern, Textilhilfsmitteln und Absorptionsmitteln für (saure) Gase.

Nicht-lineare Amine im Produktgemisch der Ethylenamine und Ethanolamine und insbesondere zyklische Amine, überwiegend Piperazin und Piperazinderivate, sind dagegen weniger begehrt bis unerwünscht.

Zur Herstellung von Ethylenaminen sind in der Literatur zahlreiche Verfahren beschrieben.

Gemäß PEP Report No. 138, "Alkyl Amines", SRI International, 03/1981, insbesondere Seiten 7, 8 13-16, 43-107, 113, 117, liefert die Umsetzung von Dichlorethan mit Ammoniak bei Molverhältnissen von 1:15 DETA mit einem Anteil an den gebildeten Ethylenaminen von größer 20 Gew.-%. Neben 40 Gew.-% EDA fallen jedoch 40 Gew.-% höhere Ethylenamine an.

Durch Aminierung von Monoethanolamin (im Folgenden: MEOA) mit Ammoniak (vgl. zum Beispiel den oben genannten PEP Report, US 4,014,933 (BASF AG)) kann die Bildung dieser höheren Ethylenamine (d. h. Ethylenaminen mit einem Siedepunkt über dem von Triethylentetramin (im Folgenden: TETA)) zugunsten von EDA weitgehend zurückgedrängt werden. Als Nebenprodukte fallen jedoch bei dieser Umsetzung Aminoethylethanolamin (im Folgenden: AEEA) und Piperazin (im Folgenden: PIP) an.

Ind. Eng. Chem. Prod. Res. Dev. 1981, 20, Seiten 399-407, (C.M. Barnes et al.) beschreibt die Ammonolyse von MEOA zu EDA an Nickel-Katalysatoren auf einem $SiO_2$-$Al_2O_3$-Mischträger. Zusatz von Wasser und der gepulverte Katalysator waren angeblich vorteilhaft bei der Erhöhung der Ausbeute an EDA.

Nachteile dieser Technologien ergeben sich bei der Suspensionskatalyse u. a. aus der notwendigen Katalysator/ Produkt Abtrennung. Darüber hinaus sind die Selektivitäten, z. B. für die Bildung von DETA, verbesserungswürdig.

WO-A-05/014623 (BASF AG) betrifft ein Verfahren zur Herstellung von Ethylenaminen (z. B. DETA) durch Umsetzung von MEOA mit Ammoniak in Gegenwart eines Katalysators in einem Reaktor (1) und Auftrennung des resultierenden Reaktionsaustrags, wobei bei der Auftrennung erhaltenes EDA in einem separaten Reaktor (2) in Gegenwart eines Katalysators zu DETA umgesetzt und der resultierende Reaktionsaustrag der Auftrennung des aus Reaktor 1 resultierenden Reaktionsaustrags zugeführt wird.

Die ältere deutsche Patentanmeldung Nr. 102005019373.0 vom 26.04.05 (BASF AG) betrifft ein Verfahren zur Herstellung von Ethylenaminen, in dem man in einer ersten Reaktionsstufe Ethylenoxid mit Ammoniak unter wasserfreien Bedingungen an einem anorganischen Ionenaustauscher kontinuierlich umsetzt, wobei das resultierende Umsetzungsprodukt Monoethanolamin, Diethanolamin und Triethanolamin in einem bestimmten Gewichtsverhältnis enthält, und das Umsetzungsprodukt anschließend kontinuierlich in einer zweiten Reaktionsstufe mit Ammoniak in Gegenwart von Wasserstoff und einem Hydrierkatalysator umsetzt.

Das Verfahren hat jedoch insbesondere den Nachteil, dass von Ethylenoxid hoher Reinheit ausgegangen werden muss.

Aus der DE 102005047464.0 ist es bekannt, die nicht zyklischen Amine, insbesondere EDA und DETA in hohen Ausbeuten und Selektivitäten durch hydrierende Aminierung ausgehend von MEOA zu erhalten, indem ein Heterogenkatalysator, enthaltend Verbindungen des Aluminiums, Kupfers, Nickels und Kobalts, eingesetzt wird, aufweisend eine Geometrie der Katalysatorformkörper entsprechend der nachfolgenden Definition:

Die Katalysatorformkörper sollen bei Kugel- oder Strangform jeweils einen Durchmesser von < 3 mm, bei Tablettenform eine Höhe von < 3 mm und bei allen anderen Geometrien jeweils einen Äquivalentdurchmesser L=1/a' von < 0,70 mm aufweisen, wobei a' die externe Oberfläche per Volumeneinheit ($mm_s^2/mm_p^3$) ist, mit:

$$a' = \frac{Ap}{Vp}$$

wobei $A_p$ die externe Oberfläche des Katalysatorpartikels ($mm_s^2$) und $V_p$ das Volumen des Katalysatorpartikels ($mm_p^3$) ist.

Aus der WO 96/38226 sind Katalysatoren bekannt, enthaltend Rhenium, Nickel, Kobalt, Bor und Kupfer und/oder Ruthenium auf einem Trägermaterial, bei einem Gewichtsverhältnis von Nickel zu Rhenium im Bereich von 1 bis 30 und einem Gewichtsverhältnis von Nickel zu Kobalt, von Nickel zu Bor sowie von Nickel zu Kupfer und/oder Ruthenium von jeweils 1 zu 20. Der Katalysator wird zur reduktiven Aminierung von Alkanen oder Arylalkanderivaten, beispielsweise mit Ammoniak in Gegenwart von Wasserstoff, insbesondere von MEOA eingesetzt. Beispielhaft angegeben ist auch eine Vielzahl weiterer Alkanderivate als Edukte, darunter Ethylenglykol. Aus dem Dokument ist nicht zu entnehmen, dass der beschriebene Katalysator für den Verfahrensschritt der Umsetzung von Monoethylenglykol zu Monoethanolamin eine erhöhte Selektivität gegenüber den Folgereaktionen von Monoethanolamin aufweisen würde.

Aus US 4,855,505 ist ein Verfahren zur Hydroaminierung von beispielsweise Monoethylenglykol mit beispielsweise Ammoniak in Gegenwart eines Katalysators bekannt, der 4 bis 40 Gew.-% Nickel oder Kobalt und 0,1 bis 5 Gew.-% Ruthenium, das als Lösung eines Rutheniumhalogenids eingeführt wurde, auf einem porösen Metalloxidträger, enthaltend mindestens 50 Gew.-% aktiviertes Aluminiumoxid, enthält. Der Katalysator wird beispielhaft in Form von Tabletten mit einer Länge und einem Durchmesser von etwa 3 mm eingesetzt. In dem Dokument ist deutlich herausgestellt, dass Nichthalogenide, wie Rutheniumoxid, Rutheniumsulfat, Rutheniumnitrat, Rutheniumnitrosylnitrat, Rutheniumammoniumnitrat, Rutheniumacetylacetonat und Kaliumperruthenat, zwar die Aktivität eines Nickel- oder Kobaltkatalysators verstärken, ohne jedoch eine nennenswerte Verbesserung der Selektivität in organischen Hydrierungen im Vergleich zu den entsprechenden Katalysatoren ohne Zusatz von Ruthenium, zu bewirken.

In Journal of Catalysis 161, Seiten 96 bis 106 (1996) ist der Einsatz von geträgerten sowie ungeträgerten Ruthenium-Kobalt-Katalysatoren für hergestellt, ausgehend von Rutheniumhalogeniden, für Hydrierungen, insbesondere von Nitrilverbindungen, beschrieben. Das Dokument gibt jedoch keinen Hinweis auf den Einsatz von Katalysatoren, enthaltend Ruthenium und Kobalt, für die hydrierende Aminierung von Monoethylenglykol mit Ammoniak.

Aus der EP-A 0 839 575 ist ein Aminierungskatalysator bekannt, umfassend, bezogen auf das Gesamtgewicht des Katalysators mehr als 6 bis 50 Gew.-% Kobalt, Nickel oder deren Gemisch, 0,001 bis 25 Gew.-% Ruthenium, 0 bis 10 Gew.-% Kupfer und 0 bis 5 Gew,-.% Promotoren auf einem porösen Metalloxidträger, wobei der Träger nicht mit Halogenverbindungen imprägniert wird. Die Katalysatoren verursachen insbesondere keine Korrosion bei gleichzeitig guter Haltbarkeit und hoher Selektivität in Bezug auf Ethylendiamin bei der Aminierung von Monoethanolamin mit Ammoniak.

Es war demgegenüber Aufgabe der Erfindung, einen Katalysator zur Verfügung zu stellen, der in einem Verfahren zur Herstellung von Ethylenaminen und Ethanolaminen durch hydrierende Aminierung von MEG für die Umsetzung von MEOA erhöhte Selektivität gegenüber bekannten Katalysatoren aufweist und gleichzeitig einen hohen Umsatz an MEG gewährleistet.

Beispielsweise sollte der Anteil an EDA und DETA im Produktgemisch größer als 60 Gew.-% sein und der Anteil an Piperazin und Piperazinderivaten je nach Bedarf, beispielsweise auf unter 20 Gew.-% im Produktgemisch begrenzt werden können, bei einem Umsatz, bezogen auf MEG von bis zu 50 % oder auch bis zu 40 %.

Die Lösung besteht in einem Verfahren zur Herstellung von Ethylenaminen und Ethanolaminen durch hydrierende Aminierung von Monoethylenglykol mit Ammoniak in Gegenwart eines Katalysators, das dadurch gekennzeichnet ist, dass ein Katalysator mit einer Aktivmasse enthaltend Ruthenium und Kobalt und daneben kein weiteres Metall der Gruppe VIII sowie kein Metall der Gruppe IB eingesetzt wird, in Form von Katalysatorformkörpern, die bei Kugelform oder Strangform jeweils einen Durchmesser von < 3 mm, bei Tablettenform eine Höhe von < 3 mm und bei allen anderen Geometrien jeweils einen Äquivalentdurchmesser L = 1/a' von < 0,70 mm aufweisen, wobei a' die externe Oberfläche per Volumeneinheit ($mm_s^2/mm_p^3$) ist, mit:

$$a' = \frac{Ap}{Vp}$$

wobei $A_p$ die externe Oberfläche des Katalysatorformkörpers ($mm_s^2$) und $V_p$ das Volumen des Katalysatorformkörpers ($mm_p^3$) ist.

Die Oberfläche und das Volumen des Katalysatorformkörpers ergeben sich aus den geometrischen Abmessungen des Formkörpers gemäß den bekannten mathematischen Formeln.

Das Volumen kann auch nach folgender Methode berechnet werden, bei der man:

1. die innere Porosität des Formkörpers bestimmt (z. B. über Messung der Wasseraufnahme in [ml/g Kat] bei Raumtemperatur und 1 bar Gesamtdruck),
2. die Verdrängung des Formkörpers beim Eintauchen in eine Flüssigkeit (z. B. durch Gasverdrängung mittels Helium-Pyknometer) bestimmt und
3. die Summe beider Volumina bildet.

Die Oberfläche kann auch nach folgender Methode theoretisch berechnet werden, bei der man eine Umhüllende des Formkörpers definiert, deren Kurvenradien max. 5 μm beträgt (um nicht die innere Porenoberfläche durch "Eindringen" der Umhüllenden in die Poren mitzunehmen) und die den Formkörper möglichst innig berührt (keine Schnittfläche mit dem Träger). Anschaulich würde das einer sehr dünnen Folie entsprechen, die man um den Formkörper legt und dann von innen ein Vakuum anlegt, so dass sich die Folie möglichst eng um den Formkörper legt.

Das als Edukt eingesetzte MEG kann nach bekannten Verfahren, beispielsweise durch Umsetzung von Ethylenoxid mit Wasser, festgestellt werden.

Die erfindungsgemäße Umsetzung erfolgt im Allgemeinen bei einem Absolutdruck im Bereich von 1-250 bar, bevorzugt 100-200 bar, insbesondere bei 150-200 bar, und im Allgemeinen bei erhöhter Temperatur, z. B. im Temperaturbereich von 100-300°C, insbesondere 130-230°C, bevorzugt bei 150-190°C.

MEG und Ammoniak werden bevorzugt in einem Mol-Verhältnis im Bereich von 3 bis 70, insbesondere 7 bis 15 eingesetzt.

Im Allgemeinen werden im erfindungsgemäßen Verfahren die Katalysatoren in einer Form eingesetzt, die nur aus der katalytisch wirksamen Aktivmasse und gegebenenfalls einem Verformungshilfsmittel, beispielsweise Grafit oder Stearinsäure, besteht, oder als geträgerter Katalysator, d. h. die katalytisch wirksame Aktivmasse ist auf einen weitgehend inaktiven Träger aufgebracht. Bei Einsatz von geträgerten Katalysatoren beträgt der Anteil des Trägers an der Gesamtmasse des Katalysators (Aktivmasse plus Träger) bevorzugt 10 bis 90 Gew.-%.

Als Träger können alle bekannten geeigneten Träger verwendet werden, beispielsweise Aktivkohle, Siliciumcarbid oder Metalloxide. Der Einsatz von Metalloxiden ist bevorzugt. Von den Metalloxiden werden vorzugsweise Aluminiumoxid, Siliciumdioxid, Titandioxid, Zirkoniumdioxid, Ceriumdioxid, Zinkoxid, Magnesiumoxid oder deren Gemische verwendet, die gegebenenfalls mit Alkali- und/oder Erdalkalimetalloxiden dotiert sind. Besonders bevorzugt sind γ-Aluminiumoxid, Siliciumdioxid, Zirkoniumdioxid, Ceriumdioxid oder Titandioxid oder Gemische davon.

Bevorzugt ist der Träger γ-Aluminiumoxid.

Die Träger können in beliebiger Form eingesetzt werden, beispielsweise als Extrudate (in Form von Strängen), Pellets, Tabletten, Monolithe, Gewebe, Gestricke oder pulverförmig.

Die geträgerten Katalysatoren können nach allgemein bekannten Verfahren hergestellt werden. Hierzu zählt etwa das Tränken eines Trägers mit Lösungen von Verbindungen der eingesetzten Metallkomponenten. Als Lösungsmittel eignen sich alle üblichen Lösungsmittel, etwa Wasser, Methanol, Ethanol oder Aceton, vorzugsweise wird Wasser eingesetzt. Weiterhin kann der Katalysator durch gemeinsames oder sequentielles Fällen der Katalysatorkomponenten, anschließende Filtration und Waschen des Filterkuchens hergestellt werden. An das Tränken bzw. Fällen schließen sich ein Trocknungsschritt (50 bis 200°C) und ein Calcinationsschritt (200 bis 500°C) an. Die Katalysatoren werden dann reduziert bei Endtemperaturen von 200 bis 400°C und können anschließen passiviert werden, da die reduzierten Metalle pyrophor sind. Nach Einbau der Katalysatoren in den Synthesereaktor können die Katalysatoren vor Anfahren der Reaktion durch Reduktion mit Wasserstoff bei Temperaturen zwischen 100 und 300°C reaktiviert werden.

Der Katalysator enthält als Aktivmasse Ruthenium und Kobalt in elementarer Form und daneben kein weiteres Metall der Gruppe VIII sowie kein Metall der Gruppe IB.

Zur Herstellung des Katalysators können Verbindungen der genannten Metalle eingesetzt werden, bevorzugt Oxide, aber auch Nitrate, Carbonate oder Acetate. Die Metallverbindungen werden zur Fertigstellung des Katalysators vor Einsatz desselben in der hydrierenden Aminierung werden die metallischen Verbindungen des Rutheniums und Kobalts reduziert, vorzugsweise durch Behandeln mit Wasserstoff. So entsteht ein Katalysator, der die genannten Metalle in elementarer, fein verteilter Form, enthält. Hierbei beträgt der Anteil des Katalysators an Ruthenium bevorzugt zwischen 0,1 und 10 Gew.-% und der Anteil an Kobalt bevorzugt zwischen 0,1 und 50 Gew.-%, jeweils bezogen auf das Gesamtgewicht der Aktivmasse des Katalysators.

Zur Herstellung der Katalysatoren mit der genannten Zusammensetzung sind verschiedene Verfahrensweisen möglich; sie sind beispielsweise durch dem Fachmann bekannte Fällungsverfahren und bevorzugt durch Tränkungsverfahren erhältlich.

Hierbei ist es wesentlich, dass der Katalysator in Form kleiner Katalysatorformkörper eingesetzt wird. Mit kleinen Katalysatorformkörpern sind solche gemeint, deren Durchmesser bei Kugel- oder Strangform jeweils einen Durchmesser von < 3 mm, bei Tablettenform eine Höhe von < 3 mm und bei allen anderen Geometrien jeweils einen Äquivalentdurchmesser $L = 1/a'$ von < 0,70 mm aufweisen, wobei a' die externe Oberfläche per Volumeneinheit ($mm_s^2/mm_p^3$) ist, mit:

$$a' = \frac{Ap}{Vp}$$

wobei $A_p$ die externe Oberfläche des Katalysatorformkörpers ($mm_s^2$) und $V_p$ das Volumen des Katalysatorformkörpers ($mm_p^3$) ist.

Besonders bevorzugt sind Katalysatorformkörper, die bei Kugel- oder Strangform jeweils einen Durchmesser von < 2,5 mm, bei Tablettenform eine Höhe von < 2,5 mm und bei allen anderen Geometrien jeweils einen Äquivalentdurchmesser L=1/a' von < 0,65 mm aufweisen.

Im erfindungsgemäßen Verfahren sind durch die kleine spezifische Dimension der Katalysatorpartikel die Diffusionswege der Reaktanden und auch der Produkte geringer. Die mittlere Verweilzeit der Moleküle in den Poren und die Wahrscheinlichkeit einer unerwünschten Folgereaktion werden hierdurch herabgesetzt. Als Folge der definierten Verweilzeit kann damit eine erhöhte Selektivität, insbesondere in Richtung des erwünschten EDAs und DETAs, erreicht werden.

Bevorzugt liegt der Katalysator als Festbett in einem Reaktor vor. Bei dem Reaktor handelt es sich bevorzugt um einen Rohrreaktor oder Rohrbündelreaktor. Bevorzugt erfolgt die Umsetzung von MEG im Reaktor im geraden Durchgang.

Das Katalysatorbett wird bevorzugt sowohl am Eingang als am Ausgang des Reaktors von einem Inertmaterial umgeben. Als Inertmaterial können zum Beispiel Pallringe, Kugeln aus einem inerten Material (zum Beispiel Keramik, Steatit, Aluminium) eingesetzt werden.

Der Reaktor kann sowohl in Sumpf- als in Rieselfahrweise betrieben werden. Bei der bevorzugten Rieselfahrweise wird bevorzugt ein Flüssigkeitsverteiler für den Reaktorfeed am Eingang des Reaktors eingesetzt. Wenn zwei Reaktoren eingesetzt werden, können beide in Sumpf- oder Rieselfahrweise betrieben werden. Alternativ kann der erste Reaktor in Sumpf- und der zweite Reaktor in Rieselfahrweise betrieben werden, oder umgekehrt.

Zur Aufrechterhaltung der Katalysatoraktivität werden bevorzugt 0,01 - 2,00 Gew.-%, besonders bevorzugt 0,20 - 0,60 Gew.-%, Wasserstoff (bezogen auf den Reaktorfeed MEG + $NH_3$) in den Reaktor gefahren.

Im bevorzugt kontinuierlichen Betrieb werden bei einer Katalysatorbelastung WHSV (weight hourly space velocity) von 0,25 - 1,25 kg/kg*h, bevorzugt 0,4 - 1 kg/kg*h (kg MEG pro kg Kat. pro Stunde) im Umsatzbereich von 40 - 50 %, bezogen auf MEG, eine Selektivität für MEOA zwischen 10 % und 50 % und für EDA und DETA zwischen 30 % und 90 %, erreicht.

Als weitere Produkte fallen im erfindungsgemäßen Verfahren geringe Mengen an Diethanolamin, DEOA ($S_{DEOA}$, im Allgemeinen 0 - 5 Gew.-%), Triethanolamin, TEOA ($S_{TEOA}$, im Allgemeinen 0 - 2 Gew.-%) und höhere Amine ($S_{höhere\ amine}$, im Allgemeinen 2 - 12 Gew.-%) an.

Im Allgemeinen enthalten die Reaktionsrohprodukte des erfindungsgemäßen Verfahrens nur geringe Mengen an zyklischen Aminen als Reaktionsprodukte (in der Regel in Mengen < 20 Gew.-%, insbesondere < 15 Gew.-%, ganz besonders 7 bis 12 Gew.-%).

Im Allgemeinen enthalten die Reaktionsrohprodukte des erfindungsgemäßen Verfahrens nur geringe Mengen an tertiären Aminen als Reaktionsnebenprodukte (in der Regel in Mengen < 10 Gew.-%, insbesondere < 7 Gew.-%, ganz besonders 0 bis 4 Gew.-%).

Die Aufarbeitung der im erfindungsgemäßen Verfahren anfallenden Produktströme, die vor allem das besonders gewünschte EDA und DETA, aber auch Aminoethylethanolamin (AEEA), Triethylentetramin (TETA), Piperazin (PIP), N-(2-Aminoethyl)-piperazin (AE-PIP) MEOA und unumgesetztes MEG enthalten, kann nach dem Fachmann bekannten Destillationsverfahren erfolgen. (Vergl. z. B. PEP Report No. 138, "Alkyl Amines", SRI International, 03/1981, Seiten 81-99,117, und DE-A-10349059 (BASF-AG)).

Die zur destillativen Reingewinnung der einzelnen Produkte, vor allem des besonders gewünschten EDAs und DETAs, benötigten Destillationskolonnen können durch den Fachmann mit ihm geläufigen Methoden ausgelegt werden (z. B. Zahl der Trennstufen, Rücklaufverhältnis, etc.).

Die Auftrennung des aus der Umsetzung resultierenden Reaktionsaustrags erfolgt insbesondere durch mehrstufige Destillation.

Zum Beispiel erfolgt die Auftrennung des aus der Umsetzung resultierenden Reaktionsaustrags in zwei Trennsequenzen durch mehrstufige Destillation, wobei in der ersten Trennsequenz zunächst Ammoniak und gegebenenfalls vorhandener Wasserstoff abgetrennt werden und in der zweiten Trennsequenz eine Auftrennung in unumgesetztes MEG sowie MEOA, EDA, PIP, DETA, AEEA, AE-PIP, TETA und höhere Ethylenamine erfolgt.

Bei der Auftrennung des aus der Umsetzung resultierenden Reaktionsaustrags anfallender Ammoniak wird bevorzugt in die Umsetzung zurückgeführt.

Bevorzugt kann die Aminierung in Gegenwart von Wasser durchgeführt werden; die Zugabe von Wasser führt zu besseren Selektivitäten für MEOA.

In einer Ausführungsvariante können zusätzlich zu dem Katalysator mit einer Aktivmasse, enthaltend Ruthenium und Kobalt, eine oder mehrere weitere Aminierungskatalysatoren eingesetzt werden, wobei der Katalysator mit einer Aktivmasse, enthaltend Ruthenium, Kobalt und daneben keine weiteren Metalle der Gruppe VIII sowie kein Metall der Gruppe IB, in einem Anteil von mindestens 20 Gew.-% eingesetzt wird. Als weitere Aminierungskatalysatoren können hierbei bekannte Aminierungskatalysatoren, beispielsweise mit einer Aktivmasse, enthaltend Nickel, Kupfer und Kobalt, eingesetzt werden.

In der Ausführungsvariante mit einem oder mehreren weiteren Aminierungskatalysatoren können dieselben

in einem ersten Reaktorbereich oder einem ersten Reaktor eingesetzt werden, worin die hydrierende Aminierung des MEGs bis zu einem MEG-Umsatz von maximal 30 % durchgeführt wird und wobei der Katalysator mit einer Aktivmasse, enthaltend Ruthenium und Kobalt und daneben kein weiteres Metall der Gruppe VIII sowie kein Metall der Gruppe IB, in einem zweiten Reaktorbereich oder in einem zweiten Reaktor eingesetzt wird, der bei einer um mindestens 10 Grad höheren Temperatur als der erste Reaktorbereich oder der erste Reaktor betrieben wird.

Bevorzugt kann der erste Reaktor oder der erste Reaktorbereich bei einer Temperatur im Bereich von 130 bis 230°C, insbesondere von 150 bis 170°C, betrieben werden und der zweite Reaktorbereich oder der zweite Reaktor bei einer Temperatur zwischen 150 und 240°C, insbesondere zwischen 160 und 190°C.

In der Verfahrensvariante mit zwei Reaktorbereichen ist der zweite Reaktorbereich vom ersten Reaktorbereich, bevorzugt durch eine Inertmaterialschicht getrennt, deren Dicke insbesondere 30 cm betragen kann.

Die Erfindung wird im Folgenden anhand von Ausführungsbeispielen näher erläutert:

Es wurde folgender Katalysator zur Umsetzung von MEG mit Ammoniak zu Ethylenaminen und Ethanolaminen eingesetzt:

151 g $Al_2O_3$-Stränge mit einem Durchmesser von 1,5 mm wurden mit 128 ml einer wässrigen Tränklösung, die 148 g Ru-Nitrosylnitrat und 46,5 g Kobaltnitrat-6-Hydrat enthielt, unter mehrmaligem guten Durchmischen bei Raumtemperatur für 60 Minuten stehen gelassen. Anschließend wurde für 4 Stunden bei 120°C unbewegt getrocknet und für 2 Stunden bei 600°C mit 150 Uh Luft calciniert. Der Katalysator enthielt 4,1 Gew.-% metallisches Kobalt und 7,1 Gew.-% metallisches Ruthenium, jeweils bezogen auf das Gesamtgewicht des Katalysators.

Beispiel 1:

40 g des oben beschriebenen Katalysators wurden in einen Reaktor eingeführt und mit Wasserstoff aktiviert. Diese Vorgehensweise gilt unverändert auch für die Beispiele 2 bis 4.

Anschließend wurden 15,2 g/h MEG, 40,7 g/h Ammoniak und 8,2 Nl/h Wasserstoff bei einem Druck von 200 bar absolut und einer Temperatur von 180°C kontinuierlich auf den Katalysator gefahren. Gaschromatographisch wurde ein MEG-Umsatz von 53,2 % bestimmt, unter Erhalt der in der Tabelle aufgeführten Anteile an EDA, MEOA, PIP, DETA und AEEA.

Beispiel 2: (Senkung der relativen Wasserstoffmenge im Zulauf)

Es wurden 15,2 g/h MEG, 42,5 g/h Ammoniak und 2,7 Nl/h Wasserstoffe bei einem Druck von 200 bar absolut und einer Temperatur von 180°C kontinuierlich auf den wie zu Beispiel 1 beschrieben vorbereiteten Katalysator gefahren. Gaschromatographisch wurde ein MEG-Umsatz von 43,4 % bestimmt, wobei die in der nachfolgenden Tabelle aufgeführten Anteile der Komponenten EDA, DEOA, MEOA, PIP, DETA und AEEA, erhalten wurden.

Beispiel 3: (Zugabe von Wasser im Zulauf)

Es wurden 17,3 g/h MEG, 22,9 g/h Wasser, 40,9 g/h Ammoniak und 2,7 Nl/h Wasserstoff bei einem Druck von 200 bar absolut und einer Temperatur von 180°C kontinuierlich auf den Katalysator gefahren. Gaschromatographisch wurde ein MEG-Umsatz von 40,4 % bestimmt, unter Erhalt der in der nachfolgenden Tabelle angegebenen Komponenten.

Beispiel 4:

Es wurden 15,1 g/h MEG, 40,7 g/h Ammoniak und 16,4 Nl/h Wasserstoff bei einem Druck von 200 bar absolut und einer Temperatur von 170°C kontinuierlich auf den Katalysator gefahren. Der gaschromatographisch bestimmte MEG-Umsatz sowie die jeweils erhaltenen Anteile der zu Beispiel 1 aufgeführten Komponenten sind in der nachfolgenden Tabelle angegeben.

Beispiel 5: (Zwei-Reaktorenkonzept)

In einen ersten Reaktor wurden 83 g eines Kupfer, Nickel, Molybdän und Zirkoniumdioxid enthaltenden Standardkatalysators gefüllt und in einen zweiten, seriell geschalteten Reaktor 30 g des oben beschriebenen erfindungsgemäßen Katalysators. Nach Aktivierung mit Wasserstoff wurden 14,8/h MEG, 42,4 g/h Ammoniak und 14,6 Nl/h Wasserstoff bei einem Druck von 200 bar absolut kontinuierlich zugeführt. Die Temperatur im ersten Reaktor wurde isotherm auf 150°C eingestellt, im zweiten Reaktor auf 170°C. Der gaschromatographisch bestimmte MEG-Umsatz betrug 42,6 %, wobei die in der nachfolgenden Tabelle aufgeführten Anteile der zu Beispiel 1 genannten Komponenten erhalten wurden.

Vergleichsbeispiel:

Es wurden 25 g eines auf Aluminiumoxidsträngen mit einem Durchmesser von 3,5 mm geträgerten Katalysators, der neben Ruthenium und Kobalt auch Kupfer und Nickel enthielt, in den Reaktor gefüllt und mit Wasserstoff aktiviert. Anschließend wurden 15,0 g/h MEG, 40,4 g/h Ammoniak und 16,4 Nl/h Wasserstoff bei einem Druck von 200 bar absolut und einer Temperatur von 200°C kontinuierlich auf den Katalysator gefahren. Gaschromatographisch wurde ein MEG-Umsatz von nur 34,4 % bestimmt, wobei 51,3 Gew.-% EDA, 17,1 Gew.% MEOA, 16,6 Gew.-% PIP, 7,3 Gew.-% DETA, 2,6 Gew.-% AEEA, 0,3 Gew.-% Diethanolamin (DEOA) und 4,8 Gew.-% andere Komponenten (Rest) erhalten wurden.

Die Versuchsergebnisse sind in der nachfolgenden Tabelle zusammengefasst:

| Beispiel | Temperatur (°C) | Druck (bar) | $H_2$(mol $H_2$/mol MEG) | Belastung (kg/l*h) | MV ($NH_3$/MEG) | Umsatz MEG % |
|---|---|---|---|---|---|---|
| 1 | 180 | 200 | 1,5 | 0,25 | 10 | 53,2 |
| 2 | 180 | 200 | 0,5 | 0,25 | 10 | 43,4 |
| 3 | 180 | 200 | 0,5 | 0,25 | 10 | 40,4 |
| 4 | 170 | 200 | 3,0 | 0,37 | 10 | 35,7 |
| 5 | 150-170 | 200 | 0,6 | 0,10 | 15 | 42,6 |
| Vergleichsbeispiel | 200 | 200 | 3,0 | 0,12 | 10 | 34,4 |

| Beispiel | EDA % | MEOA % | PIP % | DETA % | AEEA % | DEOA % | Rest % | EDA/ PIP |
|---|---|---|---|---|---|---|---|---|
| 1 | 47,2 | 20,0 | 13,3 | 5,7 | 6,7 | 1,3 | 5,7 | 3,6 |
| 2 | 57,0 | 21,9 | 8,6 | 4,2 | 4,6 | 0,6 | 3,1 | 6,6 |
| 3 | 55,5 | 26,8 | 6,3 | 3,7 | 4,5 | 0,8 | 2,4 | 8,8 |
| 4 | 30,1 | 41,6 | 6,5 | 3,3 | 8,8 | 5,2 | 4,4 | 4,6 |
| 5 | 49,5 | 29,0 | 6,8 | 3,9 | 5,7 | 1,7 | 3,4 | 7,3 |
| Vergleichsbeispiel | 51,3 | 17,1 | 16,6 | 7,3 | 2,6 | 0,3 | 4,8 | 3,1 |

Die Versuchsergebnisse zeigen, dass bei Verwendung des erfindungsgemäßen Katalysators (Beispiele 1 - 5) das EDA/PIP-Verhältnis gegenüber dem Vergleichsbeispiel erhöht ist. Die bekannte Abhängigkeit desselben von MEG-Umsatz erklärt das relativ schlechtere Ergebnis in Beispiel 1 gegenüber den weiteren Beispielen.

**Patentansprüche**

1. Verfahren zur Herstellung von Ethylenaminen und Ethanolaminen durch hydrierende Aminierung von Monoethylenglykol mit Ammoniak in Gegenwart eines heterogenen Katalysators, **dadurch gekennzeichnet, dass** ein Katalysator mit einer Aktivmasse, enthaltend Ruthenium und Kobalt und daneben kein weiteres Metall der Gruppe VIII sowie kein Metall der Gruppe IB, eingesetzt wird, in Form von Katalysatorformkörpern, die bei Kugelform oder Strangform jeweils einen Durchmesser von < 3 mm, bei Tablettenform eine Höhe von < 3 mm und bei allen anderen Geometrien jeweils einen Äquivalentdurchmesser L = 1/a' von < 0,70 mm aufweisen, wobei a' die externe Oberfläche per Volumeneinheit ($mm_s^2$/$mm_p^3$) ist, mit:

$$a' = \frac{Ap}{Vp}$$

wobei $A_p$ die externe Oberfläche des Katalysatorformkörpers ($mm_s^2$) und $V_p$ das Volumen des Katalysatorformkörpers ($mm_p^3$) ist.

2. Verfahren nach Anspruch 1, **dadurch gekennzeichnet, dass** der Katalysator als geträgerter Katalysator eingesetzt wird.

3. Verfahren nach Anspruch 1 oder 2, **dadurch gekennzeichnet, dass** der Katalysator einen Anteil von 0,1 bis 10 Gew.-% an Ruthenium und einen Anteil von 0,1 bis 50 Gew.-% an Kobalt, jeweils bezogen auf das Gesamtgewicht des Katalysators, enthält.

4. Verfahren nach Anspruch 2 oder 3, **dadurch gekennzeichnet, dass** der Katalysatorträger γ-Aluminiumoxid enthält.

5. Verfahren nach Anspruch 4, **dadurch gekennzeichnet, dass** der Katalysatorformkörper bei Kugel- oder Strangform jeweils einen Durchmesser von < 2,5 mm, bei Tablettenform eine Höhe von < 2,5 mm und bei allen anderen Geometrien jeweils einen Äquivalentdurchmesser L=1/a' von < 0,65 mm aufweist.

6. Verfahren nach einem der Ansprüche 1 bis 5, **dadurch gekennzeichnet, dass** die Aminierung in Gegenwart von Wasser durchgeführt wird.

7. Verfahren nach einem der Ansprüche 1 bis 6, **dadurch gekennzeichnet, dass** 0,01 bis 1,00 Gew.-% Wasserstoff, bevorzugt 0,20 bis 0,60 Gew.-% Wasserstoff, bezogen auf das Gesamtgewicht an dem Reaktor zugeführten Monoethylenglykol und Ammoniak, eingesetzt wird.

8. Verfahren nach einem der Ansprüche 1 bis 7, **dadurch gekennzeichnet, dass** zusätzlich zu dem Katalysator mit einer Aktivmasse, enthaltend Ruthenium und Kobalt und daneben kein weiteres Metall der Gruppe VIII sowie kein Metall der Gruppe IB, ein oder mehrere weitere Aminierungskatalysatoren eingesetzt werden, wobei der Katalysator mit einer Aktivmasse, enthaltend Ruthenium und Kobalt und daneben kein weiteres Metall der Gruppe VIII sowie kein Metall der Gruppe IB, in einem Anteil von mindestens 20 Gew.-% eingesetzt wird.

9. Verfahren nach Anspruch 8, **dadurch gekennzeichnet, dass** der oder die weiteren Aminierungskatalysatoren in einem ersten Reaktorbereich oder einem ersten Reaktor eingesetzt werden, worin die Aminierung bis zu einem Monoethylenglykol-Umsatz von maximal 30 % durchgeführt wird und der Katalysator mit einer Aktivmasse, enthaltend Ruthenium und Kobalt und daneben kein weiteres Metall der Gruppe VIII sowie kein Metall der Gruppe IB, in einem zweien Reaktorbereich oder in einem zweiten Reaktor eingesetzt wird, der bei einer um mindestens 10 °C höheren Temperatur als der erste Reaktorbereich oder der erste Reaktor betrieben wird.

10. Verfahren nach Anspruch 9, **dadurch gekennzeichnet, dass** der erste und der zweite Reaktorbereich durch eine Inertmaterialschicht voneinander getrennt sind.

11. Verfahren nach Anspruch 10, **dadurch gekennzeichnet, dass** die Dicke der Inertmaterialschicht 30 cm beträgt.

**Claims**

1. A process for preparing ethylene amines and ethanolamines by hydrogenative amination of monoethylene glycol by means of ammonia in the presence of a heterogeneous catalyst, wherein a catalyst having an active composition comprising ruthenium and cobalt and no further additional metal of group VIII and also no metal of group IB is used in the form of shaped catalyst bodies which in the case of a spherical shape or rod shape in each case have a diameter of < 3 mm, in the case of a pellet shape have a height of < 3 mm and in the case of all other geometries in each case have an equivalent diameter L = 1/a' of < 0.70 mm, where a' is the external surface area per unit volume ($mm_s^2/mm_p^3$) and:

$$a' = \frac{Ap}{Vp}$$

where Ap is the external surface area of the shaped catalyst body ($mm_s^2$) and $V_p$ is the volume of the shaped catalyst body ($mm_p^3$).

**2.** The process according to claim 1, wherein the catalyst is used as a supported catalyst.

**3.** The process according to claim 1 or 2, wherein the catalyst comprises a proportion of from 0.1 to 10% by weight of ruthenium and a proportion of from 0.1 to 50% by weight of cobalt, in each case based on the total weight of the catalyst.

**4.** The process according to claim 2 or 3, wherein the catalyst support comprises γ-aluminum oxide.

**5.** The process according to claim 4, wherein the shaped catalyst body in the case of a spherical or rod shape in each case has a diameter of < 2.5 mm, in the case of a pellet shape has a height of < 2.5 mm and in the case of all other geometries in each case has an equivalent diameter L = 1/a' of < 0.65 mm.

**6.** The process according to any of claims 1 to 5, wherein the amination is carried out in the presence of water.

**7.** The process according to any of claims 1 to 6, wherein from 0.01 to 1.00% by weight of hydrogen, preferably from 0.20 to 0.60% by weight of hydrogen, based on the total weight of the monoethylene glycol and ammonia fed to the reactor, is used.

**8.** The process according to any of claims 1 to 7, wherein one or more further amination catalysts are used in addition to the catalyst having an active composition comprising ruthenium and cobalt and no additional metal of group VIII and no metal of group IB, with the catalyst having an active composition comprising ruthenium, cobalt and no further addition metal of group VIII and no metal of group IB being used in a portion of at least 20% by weight.

**9.** The process according to claim 8, wherein the further amination catalyst or catalysts is/are used in a first reactor region or a first reactor in which the amination is carried out to a monoethylene glycol conversion of not more than 30% and the catalyst having an active composition comprising ruthenium and cobalt and no further additional metal of group VIII and no metal of group IB is used in a second reactor region or in a second reactor which is operated at a temperature which is at least 10°C higher than that in the first reactor region or the first reactor.

**10.** The process according to claim 9, wherein the first reactor region and the second reactor region are separated from one another by a bed of inert material.

**11.** The process according to claim 10, wherein the thickness of the bed of inert material is 30 cm.

**Revendications**

**1.** Procédé de fabrication d'éthylène-amines et d'éthanol-amines par amination hydrogénante de monoéthylène glycol avec de l'ammoniac en présence d'un catalyseur hétérogène, **caractérisé en ce qu'**un catalyseur comprenant une masse active contenant du ruthénium et du cobalt et, en outre, aucun métal supplémentaire du groupe VIII et aucun métal du groupe IB est utilisé, sous la forme de corps moulés de catalyseur qui présentent, sous forme sphérique ou sous forme filamentaire, à chaque fois un diamètre < 3 mm, sous la forme de tablettes, une hauteur < 3 mm et, pour toutes les autres géométries, à chaque fois un diamètre équivalent L = 1/a' < 0,70 mm, a' étant la surface extérieure par unité de volume ($mm_s^2/mm_p^3$), avec:

$$a' = \frac{Ap}{Vp}$$

Ap étant la surface extérieure du corps moulé de catalyseur ($mm_s^2$) et $V_p$ étant le volume du corps moulé de catalyseur ($mm_p^3$).

**2.** Procédé selon la revendication 1, **caractérisé en ce que** le catalyseur est utilisé sous la forme d'un catalyseur supporté.

**3.** Procédé selon la revendication 1 ou 2, **caractérisé en ce que** le catalyseur contient une proportion de 0,1 à 10 % en poids de ruthénium et une proportion de 0,1 à 50 % en poids de cobalt, à chaque fois par rapport au poids total du catalyseur.

**4.** Procédé selon la revendication 2 ou 3, **caractérisé en ce que** le support de catalyseur contient de l'oxyde d'aluminium γ.

**5.** Procédé selon la revendication 4, **caractérisé en ce que** le corps moulé de catalyseur présente, sous forme sphérique ou filamentaire, à chaque fois un diamètre < 2,5 mm, sous la forme de tablettes, une hauteur < 2,5 mm et, pour toutes les autres géométries, à chaque fois un diamètre équivalent L = 1/a' < 0,65 mm.

**6.** Procédé selon l'une quelconque des revendications 1 à 5, **caractérisé en ce que** l'amination est réalisée en présence d'eau.

**7.** Procédé selon l'une quelconque des revendications 1 à 6, **caractérisé en ce que** 0,01 à 1,00 % en poids d'hydrogène, de préférence 0,20 à 0,60 % en poids d'hydrogène, est utilisé par rapport au poids total de monoéthylène glycol et d'ammoniac introduits dans le réacteur.

**8.** Procédé selon l'une quelconque des revendications 1 à 7, **caractérisé en ce qu'**en plus du catalyseur comprenant une masse active contenant du ruthénium et du cobalt et, en outre, aucun métal supplémentaire du groupe VIII et aucun métal du groupe IB, un ou plusieurs catalyseurs d'amination supplémentaires sont utilisés, le catalyseur comprenant une masse active contenant du ruthénium et du cobalt et, en outre, aucun métal supplémentaire du groupe VIII et aucun métal du groupe IB étant utilisé en une proportion d'au moins 20 % en poids.

**9.** Procédé selon la revendication 8, **caractérisé en ce que** le ou les catalyseurs d'amination supplémentaires sont utilisés dans une première zone de réacteur ou un premier réacteur où l'amination est réalisée jusqu'à une conversion de monoéthylène glycol d'au plus 30 %, et le catalyseur comprenant une masse active contenant du ruthénium et du cobalt et, en outre, aucun métal supplémentaire du groupe VIII et aucun métal du groupe IB est utilisé dans une seconde zone de réacteur ou un second réacteur, qui est exploité à une température au moins 10 °C supérieure à celle de la première zone de réacteur ou du premier réacteur.

**10.** Procédé selon la revendication 9, **caractérisé en ce que** la première et la seconde zone de réacteur sont séparées l'une de l'autre par une couche de matériau inerte.

**11.** Procédé selon la revendication 10, **caractérisé en ce que** l'épaisseur de la couche de matériau inerte est de 30 cm.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4014933 A
- WO 05014623 A
- DE 102005019373
- DE 102005047464

- WO 9638226 A
- US 4855505 A
- EP 0839575 A
- DE 10349059 A

**In der Beschreibung aufgeführte Nicht-Patentliteratur**

- Alkyl Amines. *SRI International,* Marz 1981, 7, 8 13-1643-107113, 117

- **C.M. BARNES.** *Ind. Eng. Chem. Prod. Res. Dev.,* 1981, vol. 20, 399-407
- *Journal of Catalysis,* 1996, vol. 161, 96-106